(19)

Europäisches
Patentamt
European
Patent Office
Office européen
des brevets

(11) **EP 4 647 010 A1**

(12) **EUROPEAN PATENT APPLICATION**
published in accordance with Art. 153(4) EPC

(43) Date of publication:
**12.11.2025 Bulletin 2025/46**

(21) Application number: **24738674.1**

(22) Date of filing: **02.01.2024**

(51) International Patent Classification (IPC):
*A61B 5/346* (2021.01)        *A61B 5/00* (2006.01)
*G16H 50/20* (2018.01)        *G16H 50/30* (2018.01)
*G16H 50/50* (2018.01)        *G06N 3/08* (2023.01)

(52) Cooperative Patent Classification (CPC):
A61B 5/00; A61B 5/346; G06N 3/08; G16H 50/20;
G16H 50/30; G16H 50/50; A61B 6/503

(86) International application number:
**PCT/KR2024/000047**

(87) International publication number:
**WO 2024/147603 (11.07.2024 Gazette 2024/28)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.01.2023  KR 20230000440
28.12.2023  KR 20230194390**

(71) Applicant: **Seoul National University Hospital
Seoul 03080 (KR)**

(72) Inventors:
 • **KIM, Joonghee
  Seongnam-si, Gyeonggi-do 13539 (KR)**

 • **CHO, Youngjin
  Seongnam-si, Gyeonggi-do 13620 (KR)**
 • **CHO, Goo-Yeong
  Seongnam-si, Gyeonggi-do 13620 (KR)**
 • **YOON, Yeonyee E.
  Seongnam-si, Gyeonggi-do 13620 (KR)**
 • **HWANG, In-Chang
  Seongnam-si, Gyeonggi-do 13620 (KR)**
 • **CHOI, Hong-Mi
  Seongnam-si, Gyeonggi-do 13620 (KR)**

(74) Representative: **V.O.
P.O. Box 87930
2508 DH Den Haag (NL)**

(54) **ARTIFICIAL-INTELLIGENCE-BASED DEVICE AND METHOD FOR EVALUATING CARDIAC STRAIN THROUGH ELECTROCARDIOGRAPHY**

(57)    Embodiments provide an artificial-intelligence-based device and method for evaluating cardiac strain through electrocardiography, the device comprising: an acquisition unit for acquiring electrocardiography data of a subject; an ECG encoder which inputs with the electrocardiography data so as to extract an ECG embedding vector including feature information of the electrocardiography data; and a cardiac strain task processing unit which inputs with the ECG embedding vector so as to include and generate cardiac strain information about one or more task items. Since the electrocardiography data is input into an artificial-intelligence neural network so as to evaluate the degree of cardiac strain, various cardiac strain can be measured and a consistent quantitative assessment can be performed at a low cost.

FIG. 2

EP 4 647 010 A1

## Description

## Technical Field

The present application relates to an artificial-intelligence-based device and an artificial-intelligence-based method for evaluating cardiac strain through electrocardiography, wherein electrocardiography data is input into an artificial-intelligence neural network to quantify and evaluate the degree of cardiac strain.

## Cross-Reference to Related Applications

The present application claims priority to Korean Patent Application No. 10-2023-0000440, filed on January 2, 2023, and Korean Patent Application No. 10-2023-0194390, filed on December 28, 2023, the disclosures of which are incorporated by reference herein in their entirety.

## Background Art

Cardiac strain is a more sensitive indicator than ejection fraction (EF) in evaluating cardiac functions. The EF shows the amount of blood in percentage that the left ventricle of the heart pushes out with each contraction, while cardiac strain evaluates the contraction and relaxation capacity of the heart muscle in more detail. This may help detect early stages of heart disease or more accurately assess the degree of cardiac dysfunction. In addition, the EF is useful in evaluating the overall function of the heart, while the cardiac strain is more effective in identifying specific changes in the heart muscle.

However, the cardiac strain can only be measured by using echocardiography. The echocardiography is expensive and difficult to measure consistently due to the influence of the medical staff performing the echocardiography, the companies that manufacture echocardiography-related equipment, and the equipment.

## Detailed Description of the Invention

## Technical Solution

In order to solve the above problem, the present application uses electrocardiography (ECG) instead of evaluating cardiac strain using echocardiography.

ECG is a recording of the electrical activity of the myocardium as the heart beats. ECG is a very simple test, immediately confirms the test result, and is also useful when continuously monitoring a patient.

The present application, using ECG-based artificial intelligence, allows quantitative assessment of cardiac strain with ECG alone without echocardiography. This helps medical staff to easily and repeatedly measure cardiac strain in patients at a lower cost and in a more consistent manner. Accordingly, the present application can more sensitively screen for newly occurring cardiac dysfunctions and thus can be usefully utilized in the field of monitoring, e.g., cardiotoxicity of anticancer drugs.

## Summary of the Invention

According to embodiments of the present application, there is provided an artificial-intelligence-based device for evaluating cardiac strain through electrocardiography, the device including: an acquisition unit for acquiring electrocardiography data of a subject; an ECG encoder which inputs the electrocardiography data so as to extract an ECG embedding vector including feature information of the electrocardiography data; and a cardiac strain task processing unit which inputs the ECG embedding vector so as to include and generate cardiac strain information about one or more task items.

In one embodiment, the electrocardiography data is electrocardiography image data or time series data, wherein the time series data may be time series data corresponding to a waveform signal or an image showing the same in a two-dimensional plane, and the electrocardiography image data may be photographed image data, screen-captured image data, or image data obtained from an electronic medical record system.

In one embodiment, the cardiac strain task processing unit may input a vector obtained by concatenating the ECG embedding vector with an additional information vector prepared using a vector obtained by embedding structured data, such as the subject's age, gender, height, body weight, vital signs, presence or absence of an underlying disease, test results, presence or absence of symptoms, and presence or absence of abnormal findings in examinations, and unstructured data, such as chest X-ray images, past electrocardiography data, and natural language medical records.

In one embodiment, the cardiac strain task processing unit includes a plurality of fully connected layers, each of which may have its own size and configuration for each subsequent task group.

In one embodiment, the cardiac strain information generated by the cardiac strain task processing unit may include at least one or more of a first task group related to chamber-specific strain evaluation information, a second task group related to comprehensive strain evaluation information for the entire heart, and a third task group related to presence or absence of pathological conditions associated with cardiac strain.

In one embodiment, the chamber-specific strain evaluation information may be a chamber-specific strain measurement or strain rate measurement of a myocardial segmentation model based on an anatomical structure of the heart.

In one embodiment, the strain measurement is calculated by the following Equation 1.

[Equation 1]

$$\text{Strain} = (L - L_0) / L_0 * 100$$

The $L_0$ may represent the original length of the myocardium, and the L may represent the strained length of the myocardium.

In one embodiment, the strain rate measurement is calculated by the following Equation 2.

[Equation 2]

$$\text{Strain rate} = (V_1 - V_2) / d$$

The $V_1$ and $V_2$ may represent the speeds at which the myocardium moves from two particular points, and the d may represent the distance between the two particular points.

In one embodiment, the comprehensive strain evaluation information for the entire heart includes at least one of a left ventricular global longitudinal strain (LV-GLS), a right ventricular GLS (RV-GLS), and a peak atrial longitudinal strain (PALS). The GLS indicates a degree of longitudinal shortening of the LV-GLS or the RV-GLS. The PALS may be obtained by measuring the longitudinal strain based on the atrial maximum expansion in a left atrium (LA) reservoir phase or a right atrium (RA) reservoir phase.

In one embodiment, the presence or absence of pathological conditions associated with cardiac strain may be defined as abnormal when a specific strain measurement is lower than a predetermined threshold and may be defined as normal when the specific strain measurement is higher than the threshold.

In one embodiment, the cardiac strain information generated by the cardiac strain task processing unit may further include numerical clinical information including an echocardiography-related measurement, and an auxiliary task group predicting the presence or absence of specific pathological conditions other than cardiac strain.

In one embodiment, the device may further include a result output unit for outputting cardiac strain information generated by the cardiac strain task processing unit. The result output unit may output the chamber-specific strain evaluation information by outputting the chamber-specific strain measurement or strain rate measurement of the myocardial segmentation model based on the anatomical structure of the heart and coloring each chamber according to a numerical range of the strain measurement.

In one embodiment, the result output unit may output a probability or a score indicating a degree of abnormality or may plot the probability or the score.

In one embodiment, the result output unit may show changes in cardiac strain over time in the form of an explanatory text, a longitudinal change, a table, or a graph.

In one embodiment, the device may further include a preprocessing unit that denoises the electrocardiography data using at least one of a low-pass filter, a high-pass filter, or a notch filter before the electrocardiography data is input to the ECG encoder.

In one embodiment, the cardiac strain task processing unit is trained using a plurality of learning datasets, wherein the plurality of learning datasets may utilize echocardiography test results for which cardiac strain evaluation is completed and electrocardiography data pairs obtained by the same person within a predetermined time frame.

In one embodiment, the cardiac strain task processing unit may train a subset of a plurality of task groups using multitask learning and may be optimized as hyperparameters for the proportion of loss for each task group and the proportion of loss for each task within each task group.

According to another embodiment of the present application, there is provided an artificial-intelligence-based method for evaluating cardiac strain through electrocardiography performed by a processor, the method including: acquiring electrocardiography data of a subject; inputting the electrocardiography data so as to extract an ECG embedding vector including feature information of the electrocardiography data; and inputting the ECG embedding vector so as to include and generate cardiac strain information about one or more task items.

In one embodiment, the method may further include a preprocessing step of denoising the electrocardiography data using at least one of a low-pass filter, a high-pass filter, or a notch filter, prior to extracting the ECG embedding vector.

In one embodiment, the cardiac strain information may include at least one or more of a first task group related to chamber-specific strain evaluation information, a second task group related to comprehensive strain evaluation information for the entire heart, and a third task group related to presence or absence of pathological conditions associated with cardiac strain.

According to other exemplary embodiments of the present application, there is provided a computer-readable recording medium readable by a computer and storing program instructions operable by the computer, wherein the program instructions, when executed by a processor of the computer, cause the processor to perform the artificial-intelligence-based method of evaluating cardiac strain through electrocardiography.

**Effect of the Invention**

The present application allows various cardiac strain to be measured by inputting electrocardiography data into an artificial-intelligence neural network to evaluate the degree of cardiac strain and enables consistent quantitative assessment at a low cost.

This helps medical staff to provide emergency care with higher confidence in diagnosis, thereby improving patient safety.

The effects of the present application are not limited to the effects mentioned above, and other effects not mentioned will be clearly understood by those skilled in the art from the description of the claims.

**Brief Description of the Drawings**

In order to more clearly describe exemplary embodiments of the present application, the drawings required for describing the embodiments are briefly introduced below. It should be understood that the following drawings are only for the purpose of describing embodiments of the present application and not for the purpose of limiting the same. In addition, for clarity of explanation, some elements may be shown in the drawings below with various modifications, such as exaggeration or omission.

FIG. 1 is a schematic diagram of an artificial-intelligence-based device for evaluating cardiac strain through electrocardiography, according to one aspect of the present application.

FIG. 2 is a schematic diagram illustrating a process of generating information on N cardiac strain task groups based on electrocardiography data, in one embodiment of the present application.

FIG. 3 is a diagram illustrating a method of calculating a quantified cardiac strain rate measurement, in one embodiment of the present application.

FIGS. 4A to 4C are diagrams illustrating a method of displaying chamber-specific strain evaluation information on a Bull's eye map, which is one of 16-segment, 17-segment and 18-segment models, in one embodiment of the present application.

FIGS. 5A to 5C are diagrams illustrating a method of coloring each chamber according to a numerical range of the strain measurement and displaying the color on the Bull's eye map, in one embodiment of the present application.

FIGS. 6A and 6B are examples of the result output unit showing longitudinal changes in cardiac strain over time in the form of a table or a graph, in one embodiment of the present application.

FIG. 7 is a flowchart of an artificial-intelligence-based method for evaluating cardiac strain through electrocardiography, in one embodiment of the present application.

**Best Mode for Carrying Out the Invention**

Hereinafter, some embodiments of the present application will be described in detail with reference to illustrative drawings. In adding reference numerals to the components in each drawing, the same components may have the same numerals as far as possible, even though they are indicated in other drawings. In addition, in the description of the present embodiments, when it is determined that the specific description of the related known configuration or function may obscure the gist of the present technical idea, the detailed description thereof may be omitted.

**Definitions**

When "comprise", "have", "consist of", and the like mentioned in the present application are used, other components can be added unless "only" is used. When the components are expressed in the singular form, it may include the plural form unless otherwise specified.

In addition, in describing the components of the present application, terms such as "first", "second", "A", "B", " (a)", and " (b)" may be used. Unless otherwise specified, these terms are only used to distinguish the components from other components, and the nature, sequence, order, or number of the components are not limited by the terms.

In the present application, "training" or "learning" is a term that refers to performing machine learning through computing according to a procedure.

It is intended that terms such as "unit," "module," "device," or "system" in the present application may refer to a combination of hardware and software driven by that hardware. For example, hardware may be a data processing device including a central processing unit (CPU), a graphic processing unit (GPU), or another processor. In addition, software may refer to a process being executed, an object, an executable, a thread of execution, a program, and the like.

A neural network in the present application refers to a machine learning algorithm or network. An encoder outputs a numerical vector that provides feature information of input data.

The Bull's eye map in the present application is a myocardial segmentation model based on the anatomical structure of the heart.

**Description of Exemplary Implementations**

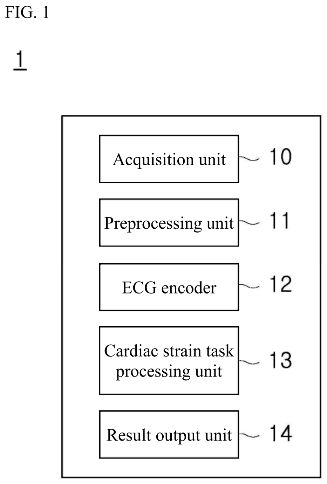

FIG. 1 is a schematic diagram of an artificial-intelligence-based device for evaluating cardiac strain through electrocardiography, according to one aspect of the present application.

Referring to FIG. 1, an artificial-intelligence-based device 1 for evaluating cardiac strain through electrocardiography ("cardiac strain evaluation device") may include an acquisition unit 10, a preprocessing unit 11, an ECG encoder 12, a cardiac strain task processing unit 13, and a result output unit 14.

The acquisition unit 10 acquires electrocardiography data of a subject. The electrocardiography or electrocardiography data is electrocardiography image data or time series data. The time series data is time series data corresponding to a waveform signal or an image showing the same in a two-dimensional plane. The electrocardiography image data may be photographed image data, screen-captured image data, or image data obtained from an electronic medical record system. The electrocardiography data may include at least one or more of electrocardiography signals that are one-dimensional single-channel or multi-channel signals and electrocardiography images in which the electrocardiography signals are shown on a two-dimensional plane. In one embodiment, the signals may be, for example, in the form of a two-dimensional array of C×T (the number of each input lead (channel) × the number of measurement values for each channel), and the images may be images including all lead channels or images including patch images for each lead channel cropped for each lead channel. This may be in the form of a two-dimensional array of single-channel black and white images (W×H; the number of horizontal pixels × the number of vertical pixels) or in the form of a three-dimensional array of color images (C×W×H; the number of color channels × the number of horizontal pixels × the number of vertical pixels) having three channels of red (R), green (G), and blue (B).

However, the present application is not limited to the electrocardiography data. All other bio-data that is patient-derived information may be used, including various time-series bio-signals, such as electroencephalogram (EEG), electromyogram (EMG), electrooculography (EOG), and the like.

The preprocessing unit 11 denoises the electrocardiography data by using at least one of a low-pass filter, a high-pass filter, and a notch filter before the electrocardiography data is input to the ECG encoder. In addition, the preprocessing unit 11 may perform data augmentation to secure data necessary for learning of the artificial-intelligence neural network. In one example, the preprocessing unit 11 may augment and process various one-dimensional and two-dimensional electrocardiography data suitable for input data dimensions during training of the ECG encoder 12 or the cardiac strain task processing unit 13. For example, in the case of a one-dimensional signal data input method, various random changes (e.g., amplitude rescale, time shift, substitution of a specific region by 0, noise addition) may be applied to each one-dimensional lead channel, and in the case of a two-dimensional image input method, at least one or more of image resize, rotate, horizontal flipping, vertical flipping, crop transformation, noise addition, and deep learning-based GAN techniques may be used.

The ECG encoder 12 inputs the electrocardiography data to extract an ECG embedding vector including feature information of the electrocardiography data. The ECG encoder 12 is a neural network including a convolutional neural network (CNN), a recurrent neural network (RNN), a multilayer perceptron (MLP), a transformer, and the like that receives the electrocardiography data.

The cardiac strain task processing unit 13 inputs the ECG embedding vector to include and generate cardiac strain information about one or more task items.

The result output unit 14 outputs the cardiac strain information generated by the cardiac strain task processing unit 13. The result output unit 14 may include a display and may be further configured to output various cardiac strain information on the display. A user may monitor the analysis result in real time through a display screen of a smartphone, a tablet PC, and the like. The display may be selected from various output members, such as a liquid crystal display (LCD), a thin film transistor-liquid crystal display (TFT LCD), an organic light-emitting diode (OLED), a flexible display, a three-dimensional display (3D display), AR glass, and VR glass.

It will be apparent to those skilled in the art that the cardiac strain evaluation device 1 may include other components which are not described herein. For example, the cardiac strain evaluation device 1 may include other hardware elements necessary for the operations described herein, including a network interface, an input device for data entry, and an output device for display, printing, or other data presentation.

Hereinafter, detailed descriptions of the ECG encoder 12 and the cardiac strain task processing unit 13 refer to the drawings described below.

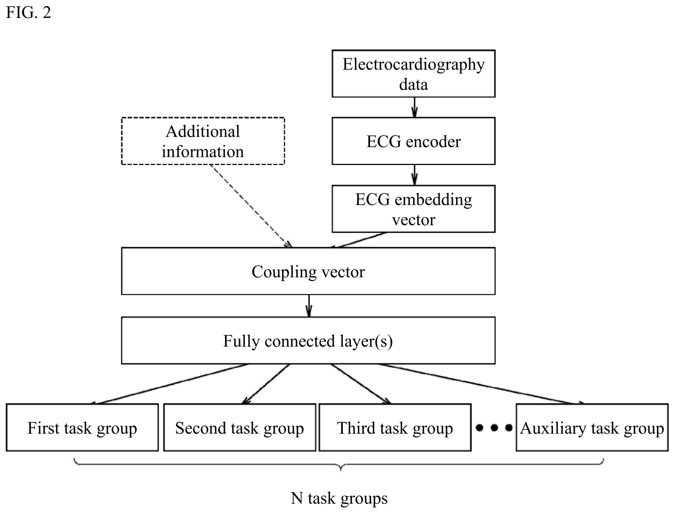

FIG. 2 is a schematic diagram illustrating a process of generating information on N cardiac strain task groups based on electrocardiography data, in one embodiment of the present application.

Referring to FIG. 2, the ECG encoder 12 inputs the electrocardiography data to extract the ECG embedding vector including feature information of the electrocardiography data. The ECG encoder 12 is a neural network including a CNN, an RNN, an MLP, a transformer, and the like that receives the electrocardiography data. The ECG encoder 12 may be any kind of artificial neural network, including the CNN, RNN, MLP, transformer, and various variations thereof. The ECG encoder 12 is not limited to a specific neural network structure. The ECG encoder 12 is used by fine-tuning, through transfer learning, a separate encoder network randomly initialized before training or developed through separate training (e.g., supervised learning, unsupervised learning, self-supervised learning).

The cardiac strain task processing unit 13 may input a vector obtained by concatenating the ECG embedding vector with an additional information vector prepared by preprocessing and integrating at least one or more of standardized test results, such as clinical information including at least one of the subject's age, gender, height, and weight, vital signs, presence or absence of an underlying disease, test results, presence or absence of symptoms, and presence or absence of specific abnormal findings during a physical examination, or at least one or more of the embedded unstructured test results including a chest X-ray image, past electrocardiography data, and natural language. Additionally, the additional information may be a numerical vector having one or more elements, including information on the presence or absence of a particular radiological finding using morphological features provided in the chest x-ray image, a parameter of a distribution function defining a numerical value, a probability or a distribution thereof utilized for a task of diagnosing or predicting a particular disease, and the like. The additional information may also include functional abnormality information associated with abnormal findings found using patient basic information and basic information based on morphological features, as well as other basic information selected depending on whether it has a medical, statistical, causal, or practical relevance. Although the additional information is not an essential component of the present application, the prediction capability can be improved when the additional information is input to the cardiac strain task processing unit 13, together with the electrocardiography data.

Although the ECG embedding vector and the coupling vector are described as vectors for ease of understanding, the ECG embedding vector and the coupling vector may take the form of an N-dimensional array. In this case, a concatenation scheme may be implemented in an array axial direction suitable for the entire network structure.

The cardiac strain task processing unit 13 includes a plurality of fully connected layers, each of which may have its own size and configuration for each subsequent task group. In one example, various modifications, such as normalization layer, dropout and similar layer, Bayesian layer, attention layer, and residual connection, may be applied to the plurality of fully connected layers.

The cardiac strain information generated by the cardiac strain task processing unit 13 may include at least one or more of a first task group related to chamber-specific strain evaluation information, a second task group related to comprehensive strain evaluation information for the entire heart, and a third task group related to the presence or absence of pathological conditions associated with cardiac strain.

The cardiac strain task processing unit 13 may train subsets of the plurality of task groups using multitask learning and may be optimized as hyperparameters for the proportion of loss for each task group and the proportion of loss for each task within each task group.

The chamber-specific strain evaluation information may be a chamber-specific strain measurement or strain rate measurement of the Bull's eye map, which is one of the 16-segment, 17-segment and 18-segment models. To this end, the neural network of the cardiac strain task processing unit 13 is trained according to a regression task such that the final output values become cardiac strain (or strain rate) measurements (or preprocessed values thereof). In this regression task, a mean-absolute loss (L1) and a mean-squared loss (L2) may be used in the loss function for the numerical prediction. However, various loss functions can be used without being limited to the loss function.

The comprehensive strain evaluation information for the entire heart includes at least one of a left ventricular-global longitudinal strain (LV-GLS), a right ventricular-GLS (RV-GLS) and a peak atrial longitudinal strain (PALS). The GLS indicates a degree of longitudinal shortening of the LV-GLS or the RV-GLS. The PALS may be obtained by measuring the longitudinal strain based on the atrial maximum expansion in a left atrium (LA) reservoir phase or a right atrium (RA) reservoir phase. The cardiac strain task processing unit 13 may directly predict the LV-GLS, the RV-GLS, the PALS, and the like, and the final output values of the neural network may correspond to the measurements, to which the regression task described above is applied. The mean-absolute loss (L1) and the mean-squared loss (L2) may be used as numerical prediction loss functions in such a regression task. However, various loss functions may be used without being limited to the loss function. The cardiac strain task processing unit 13 may predict, for each cardiac strain measurement (LV-GLS, RV-GLS, and PALS), a category of the cardiac strain measurement. The final output values of the neural network may be passed through a softmax function to output a probability corresponding to each category. In one example, the category may be classified according to the cardiac strain risk and may be represented as low, mid, or high probability. The loss function may use a categorical loss function of a cross entropy or a similar concept. In another embodiment, a predetermined range may be divided into L sections, and final output values of the neural network corresponding to the number of boundary points may be passed through a sigmoid function to obtain probabilities respectively in order to predict whether the value is greater (or less) than each section boundary point, and a final prediction value (e.g., a GLS value) may be obtained based on the probabilities. The method of acquiring the final prediction value based on the corresponding probability values varies and is not limited to a specific method. Detailed definitions and measurement methods of the GLS and PALS may vary depending on the equipment (echocardiographic instrument and analysis software).

The presence or absence of pathological conditions associated with cardiac strain may be defined as abnormal when a specific strain measurement value is lower than a predetermined threshold and may be defined as normal when it is higher than the threshold. In order to predict the presence or absence of such abnormality (or normality), the final output value of the neural network is obtained as a probability by passing through a sigmoid function and the task processing unit is trained using a cross entropy loss.

The cardiac strain information generated by the cardiac strain task processing unit 13 may further include numerical clinical information including an echocardiography-related measurement, and an auxiliary task group predicting the presence or absence of specific pathological conditions other than cardiac strain. In one embodiment, the auxiliary task group may predict numerical clinical information, such as various measurements of echocardiography (e.g., left ventricular ejection fraction), and may use L1 and L2 loss functions as numerical prediction loss functions during training. Additionally, the auxiliary task group may categorically predict the presence or absence of specific pathological conditions and may use a binary cross entropy loss or the like.

The cardiac strain task processing unit 13 may be trained using a plurality of learning datasets and the plurality of learning datasets may utilize echocardiography test results for which cardiac strain evaluation is completed and electrocardiography data pairs obtained by the same person in a predetermined time frame.

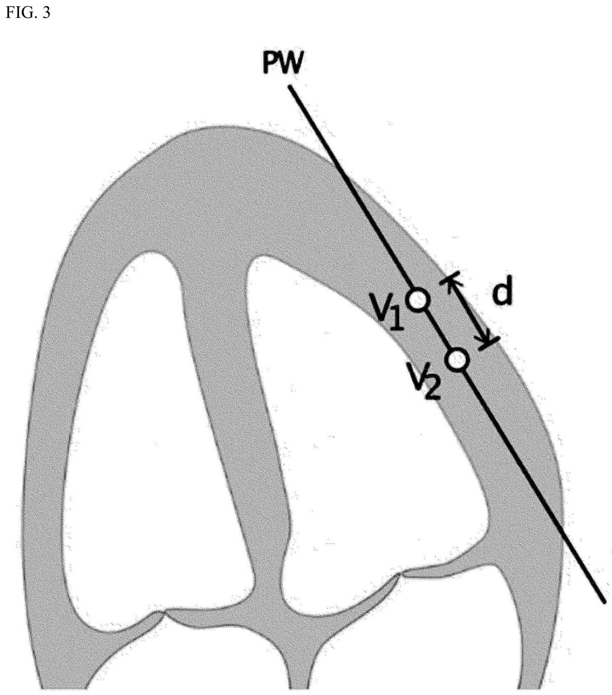

FIG. 3 is a diagram illustrating a method of calculating a quantified cardiac strain rate measurement, in one embodiment of the present application.

Referring to FIG. 3, the chamber-specific strain evaluation information may be a chamber-specific strain measurement or strain rate measurement of the Bull's eye map, which is one of the 16-segment, 17-segment and 18-segment models. The strain measurement is calculated by the following Equation 1.

$$[Equation\ 1]$$
$$Strain = (L - L_0)\ /\ L_0\ *\ 100$$

The $L_0$ may represent the original length of the myocardium and the L may represent the strained length of the myocardium.

The strain rate measurement is calculated by the following Equation 2.

$$[Equation\ 2]$$
$$Strain\ rate = (V_1 - V_2)\ /\ d$$

The $V_1$ and $V_2$ may represent the speeds at which the myocardium moves, and the d may represent the distance the myocardium has moved. For reference, the strain measurement and the strain rate measurement may be regarded as mutually equivalent.

The strain measurement or strain rate measurement means that when the absolute value increases, the degree of cardiac strain increases, and there is no problem with cardiac function. The present application allows various cardiac strain to be measured by inputting electrocardiography data into the artificial-intelligence neural network to evaluate the degree of cardiac strain and enables consistent quantitative assessment at a low cost.

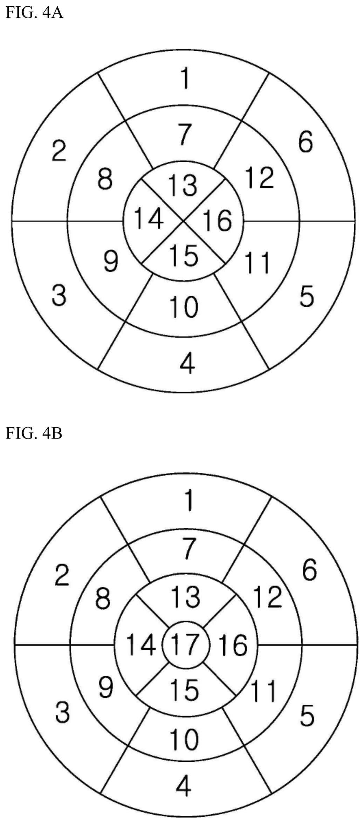

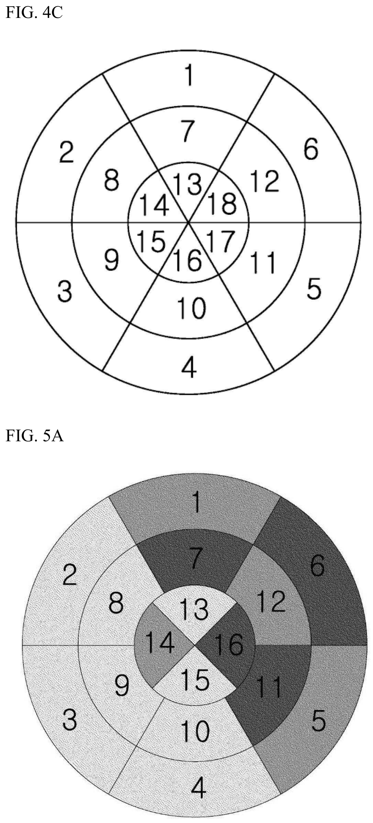

FIGS. 4A to 4C are diagrams illustrating a method of displaying chamber-specific strain evaluation information on the Bull's eye map, which is one of 16-segment, 17-segment and 18-segment models, in one embodiment of the present application.

Referring to FIGS. 4A to 4C, the Bull's eye map may include a process of splitting a region (septum) of the left ventricle to which the right ventricle is attached into two parts, and a process of splitting the other region of the left ventricle into four equal parts, and may be generated as one of the 16-segment, the 17-segment and the 18-segment models. The segments 16, 17 and 18 are not particularly limited or fixed thereto. In the case of 16-segment and 17-segment models, the numerical regions correspond to 1: basal anterior, 2: basal anteroseptal, 3: basal inferoseptal, 4: basal inferior, 5: basal inferolateral, 6: basal anterolateral, 7: mid anterior, 8: mid anteroseptal, 9: mid inferoseptal, 10: mid inferior, 11: mid inferolateral, 12: mid anterolateral, 13: apical anterior, 14: apical septal, 15: apical inferior, 16: apical lateral, and 17: apex. In the case of the 18-segment model, the numerical regions correspond to the above except for 13: apical anterior, 14; apical anteroseptal, 15: apical inferoseptal, 16: apical inferior, 17: apical inferolateral, 18: apical anterolateral. In addition, a simpler form of division is also applicable, if necessary.

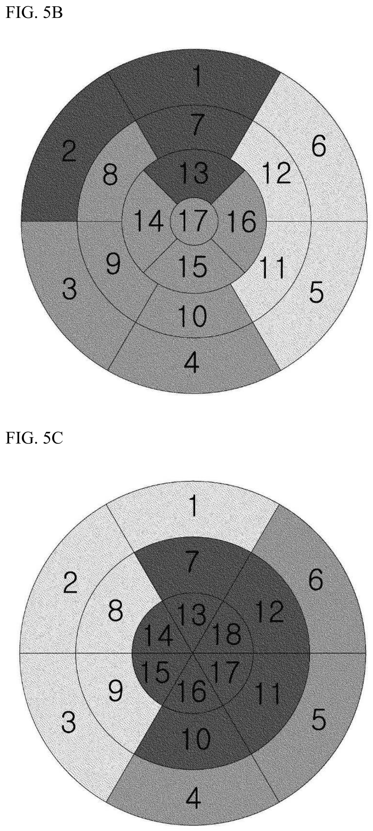

FIGS. 5A to 5C are diagrams illustrating a method of coloring each chamber according to a numerical range of the strain measurement and displaying the color on the Bull's eye map, in one embodiment of the present application.

Referring to FIGS. 5A to 5C, the result output unit 14 may output the strain measurement or the strain rate measurement for each chamber on the Bull's eye map by coloring each chamber according to a numerical range of the strain measurement. In one embodiment, the numerical range of the strain measurement may be classified into high-risk, risk, and normal categories and may be expressed in red, orange, and yellow when falling into the corresponding categories. Since the coloring method is displayed in consideration of the anatomical position of the heart, the user may intuitively grasp the degree of cardiac dysfunction, thereby improving readability.

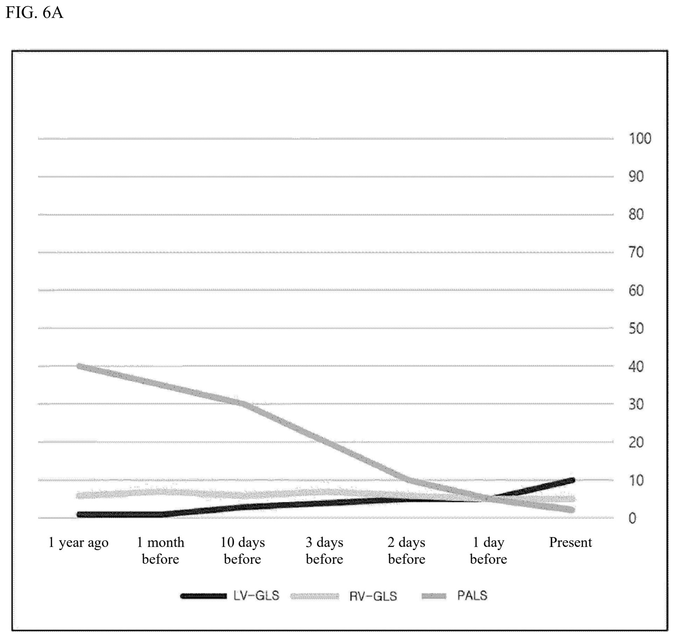

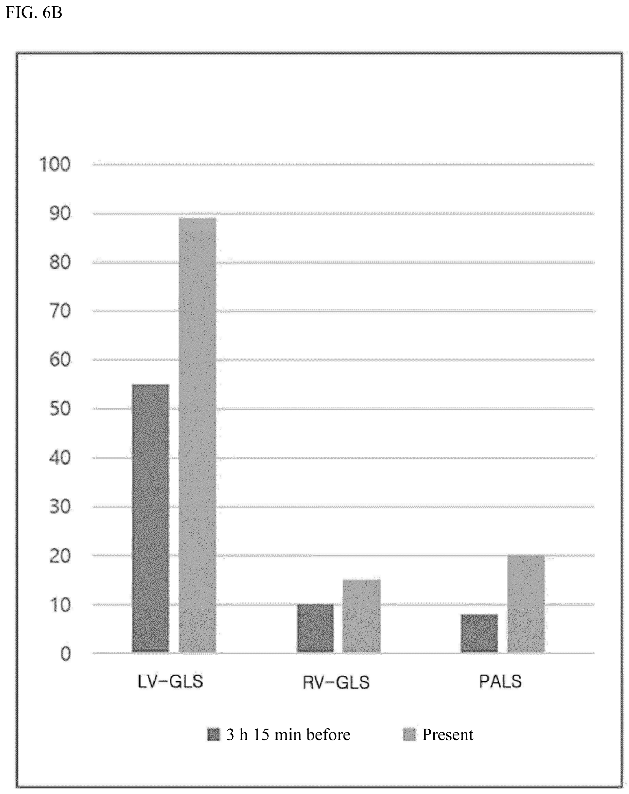

FIGS. 6A and 6B are examples of the result output unit showing longitudinal changes in cardiac strain over time in the form of a table or a graph, in one embodiment of the present application.

The result output unit 14 may output a probability or a score indicating the degree of myocardial abnormality or may plot the probability or the score. In addition, the change in cardiac strain over time may be shown in the form of an explanatory text, a longitudinal change, a table, or a graph. The results related to the comprehensive strain evaluation information for the entire heart may output the GLS or PALS values. In one example, the result output unit 14 may display and output "The calculated LV-GLS of the patient is -18%", "The probability of a serious problem (GLS < 12%) in the patient's heart function is 50%", "The low GLS score, which indicates that there is a serious problem in the patient's heart function (GLS <12%), is 50", or the corresponding probability/score as a bar plot or a box plot and a confidence interval of the output value. Referring to FIGS. 6A and 6B, when the result output unit 14 shows the change in cardiac strain over time in an explanatory text, a longitudinal change, a table, or a graph, the result output unit 14 may output "Compared to electrocardiography performed 90 days ago, the LV-GLS decreased from -20 to -10 and the contraction force decreased by 50%", may retrieve the previous analysis results and display the same as line graphs to enable multi-faceted tracking and evaluation, or may configure the time axis as a linear scale or a logarithmic non-linear scale. Additionally, the electrocardiography data at the time of the test may be analyzed and compared to test results conducted immediately before the test (e.g., 3 hours and 15 minutes).

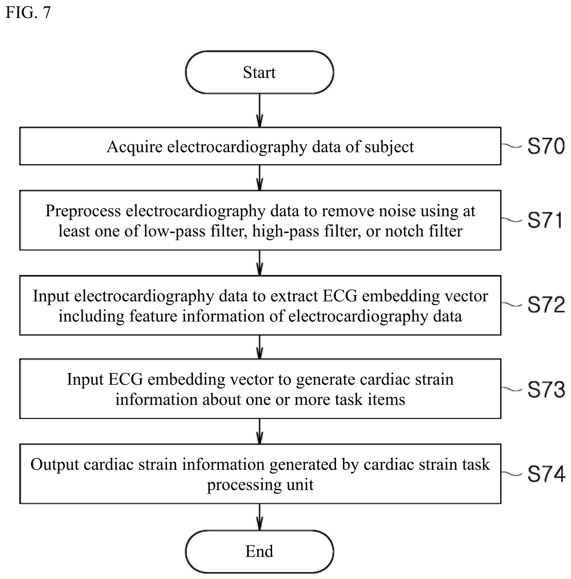

FIG. 7 is a flowchart of an artificial-intelligence-based method for evaluating cardiac strain through electrocardiography, in one embodiment of the present application.

Referring to FIG. 7, an artificial-intelligence-based method of evaluating cardiac strain through electrocardiography may include: acquiring (e.g., by the acquisition unit 10) electrocardiography data of a subject (S70); preprocessing (e.g., by the preprocessing unit 11) the electrocardiography data using at least one of a low-pass filter, a high-pass filter, or a notch filter to remove noise (S71); inputting (e.g., by the ECG encoder 12) the electrocardiography data to extract an ECG embedding vector including feature information of the electrocardiography data (S72); inputting (e.g., by the cardiac strain task processing unit 13) the ECG embedding vector to include and generate cardiac strain information about one or more task items (S73); and outputting (e.g., by the result output unit 14) the generated cardiac strain information (S74). The cardiac strain information may include at least one or more of a first task group related to chamber-specific strain evaluation information, a second task group related to comprehensive strain evaluation information for the entire heart, and a third task group related to the presence or absence of pathological conditions associated with cardiac strain. For clarity of explanation, the artificial-intelligence-based method for evaluating cardiac strain through electrocardiography has been described in more detail with embodiments performed by the cardiac strain evaluation device 1.

According to the artificial-intelligence-based device and method for evaluating cardiac strain through electrocardiography, the electrocardiography data is input to the artificial-intelligence neural network to evaluate the degree of cardiac strain, thereby allowing various cardiac strain to be measured and enabling consistent quantitative assessment at a low cost. This helps medical staff to provide emergency care with higher confidence in diagnosis, thereby improving patient safety.

The operations performed by the device according to embodiments described above may be at least partially implemented as a computer-implementable method or a computer program and may be recorded in a computer-readable recording medium. For example, the operations may be implemented with a program product consisting of a computer-readable medium including program code, which can be executed by a processor to perform any or all of the steps, operations, or processes described above.

The computer may be a computing device, such as a desktop computer, a laptop computer, a notebook computer, a smartphone, or the like, or any integrated device. The computer is a device having one or more general and special purpose processors, memory, storage, and networking components (either wireless or wired). The computer may run an operating system, such as an operating system compatible with Microsoft's Windows, Apple OS X or iOS, Linux distribution, or Google's Android OS.

The computer-readable recording medium includes all kinds of recording devices for storing data that can be read by the computer. Examples of the computer-readable recording medium include read-only memory (ROM), random-access memory (RAM), compact disc (CD)-ROM, a magnetic tape, a floppy disk, an optical data storage device, and the like. In addition, the computer-readable recording medium may be distributed across a network-connected computer system, allowing computer-readable code to be stored and executed in a distributed manner. In addition, functional programs, code, and code segments for implementing the present embodiment may be easily understood by those skilled in the art to which the present embodiment belongs.

The present application discussed above has been described with reference to the embodiments shown in the drawings, but it is merely illustrative. Those skilled in the art may understand that various modifications of embodiments are possible therefrom. However, such modifications should be considered to be within the technical protection scope of the present application. Therefore, the true technical protection scope of the present application should be determined by the technical idea of the appended claims.

**Industrial Applicability**

The present application allows quantitative assessment of cardiac strain through electrocardiography alone, without echocardiography, using electrocardiography-based artificial intelligence. This allows medical staff to easily and repeatedly measure cardiac strain from patients at a lower cost and in a more consistent manner. This may be used to more sensitively screen for newly occurring cardiac dysfunction and thus may be useful in the fields of monitoring, e.g., the cardiotoxicity of anticancer drugs.

**Claims**

1. An artificial-intelligence-based device for evaluating cardiac strain through electrocardiography, the device comprising:

an acquisition unit for acquiring electrocardiography data of a subject;
an ECG encoder which inputs the electrocardiography data so as to extract an ECG embedding vector including feature information of the electrocardiography data; and
a cardiac strain task processing unit which inputs the ECG embedding vector so as to generate cardiac strain information about one or more task items.

2. The device of claim 1, wherein the electrocardiography data is electrocardiography image data or time series data,

the time series data is time series data corresponding to a waveform signal or an image showing the time series data in a two-dimensional plane, and
the electrocardiography image data is photographed image data, screen-captured image data, or image data obtained from an electronic medical record system.

3. The device of claim 1, wherein the cardiac strain task processing unit inputs a vector obtained by concatenating the ECG embedding vector with an additional information vector prepared using a vector obtained by embedding structured data such as the subject's age, gender, height, weight, vital signs, presence or absence of an underlying disease, test results, presence or absence of symptoms, and presence or absence of abnormal findings in examinations, and unstructured data such as chest X-ray images, past electrocardiography data, and natural language medical records.

4. The device of claim 1, wherein the cardiac strain task processing unit comprises a plurality of fully connected layers, and each of the plurality of fully connected layers has its own size and configuration for each subsequent task group.

5. The device of claim 1, wherein the cardiac strain information generated by the cardiac strain task processing unit comprises at least one of a first task group related to chamber-specific strain evaluation information, a second task group related to comprehensive strain evaluation information for the entire heart, and a third task group related to presence or absence of pathological conditions associated with cardiac strain.

6. The device of claim 5, wherein the chamber-specific strain evaluation information is a chamber-specific strain measurement or strain rate measurement of a myocardial segmentation model based on an anatomical structure of the heart.

7. The device of claim 6, wherein the strain measurement is calculated by the following Equation 1:

[Equation 1]

$$\text{Strain} = (L - L_0) / L_0 * 100,$$

wherein the L0 represents the original length of the myocardium, and the L represents the strained length of the myocardium.

8. The device of claim 6, wherein the strain rate measurement is calculated by the following Equation 2:

[Equation 2]

$$\text{Strain rate} = (V_1 - V_2) / d,$$

wherein the V1 and V2 are the speeds at which the myocardium moves from two particular points, and the d represents the distance between the two particular points.

9. The device of claim 5, wherein the comprehensive strain evaluation information for the entire heart comprises at least one of a left ventricular global longitudinal strain (LV-GLS), a right ventricular GLS (RV-GLS), and a peak atrial longitudinal strain (PALS),

the GLS represents a degree of longitudinal shortening of the LV-GLS or the RV-GLS, and
the PALS measures a longitudinal strain based on atrial maximum expansion in a left atrium (LA) reservoir phase or a right atrium (RA) reservoir phase.

10. The device of claim 5, wherein the presence or absence of pathological conditions associated with cardiac strain is defined as abnormal when a specific strain measurement is lower than a predetermined threshold and is defined as normal when it is higher than the threshold.

11. The device of claim 5, wherein the cardiac strain information generated by the cardiac strain task processing unit further comprises numerical clinical information including an echocardiography-related measurement and an auxiliary task group that predicts the presence or absence of specific pathological conditions other than cardiac strain.

12. The device of claim 1, further comprising a result output unit configured to output the cardiac strain information generated by the cardiac strain task processing unit, wherein the result output unit outputs chamber-specific strain evaluation information by outputting a chamber-specific strain measurement or strain rate measurement of a myocardial segmentation model based on an anatomical structure of the heart and coloring each chamber according to a numerical range of the strain measurement.

13. The device of claim 12, wherein the result output unit outputs a probability or a score indicating a degree of abnormality or plots the probability or the score.

14. The device of claim 12, wherein the result output unit shows a change in cardiac strain over time in an explanatory text, a longitudinal change, a table, or a graph.

15. The device of claim 1, further comprising a preprocessing unit configured to denoise the electrocardiography data by using at least one of a low-pass filter, a high-pass filter, and a notch filter before the electrocardiography data is input to the ECG encoder.

16. The device of claim 1, wherein the cardiac strain task processing unit is trained using a plurality of learning datasets, the plurality of learning datasets utilizing echocardiography test results for which cardiac strain evaluation has been completed and electrocardiography data pairs obtained by the same person within a predetermined time frame.

17. The device of claim 16, wherein the cardiac strain task processing unit trains subsets of a plurality of task groups using multitask learning and is optimized as hyperparameters for the proportion of loss for each task group and the proportion of loss for each task within each task group.

18. An artificial-intelligence-based method for evaluating cardiac strain through electrocardiography performed by a processor, the method comprising:

   acquiring electrocardiography data of a subject;
   inputting the electrocardiography data so as to extract an ECG embedding vector including feature information of the electrocardiography data; and
   inputting the ECG embedding vector so as to generate cardiac strain information about one or more task items.

19. The method of claim 18, further comprising preprocessing the electrocardiography data to remove noise using at least one of a low-pass filter, a high-pass filter, or a notch filter, prior to extracting the ECG embedding vector.

20. The method of claim 18, wherein the cardiac strain information comprises at least one of a first task group related to chamber-specific strain evaluation information, a second task group related to comprehensive strain evaluation information for the entire heart, and a third task group related to presence or absence of pathological conditions associated with cardiac strain.

21. A computer-readable recording medium readable by a computer and storing program instructions operable by the computer, wherein the program instructions, when executed by a processor of the computer, cause the processor to perform the artificial-intelligence-based method for evaluating cardiac strain through electrocardiography, according to any one of claims 18 to 20.

FIG. 1

<u>1</u>

FIG. 2

```
                              ┌─────────────────────┐
                              │ Electrocardiography │
                              │       data          │
                              └─────────────────────┘
                                        │
  ┌ ─ ─ ─ ─ ─ ─ ─ ─ ─ ┐                 ▼
  │    Additional     │        ┌─────────────────────┐
  │   information     │        │     ECG encoder     │
  └ ─ ─ ─ ─ ─ ─ ─ ─ ─ ┘        └─────────────────────┘
            ╲                            │
             ╲                           ▼
              ╲                ┌─────────────────────┐
               ╲              │    ECG embedding     │
                ╲             │        vector        │
                 ╲            └─────────────────────┘
                  ╲                    ╱
  ┌──────────────────────────────────────────────────┐
  │                  Coupling vector                  │
  └──────────────────────────────────────────────────┘
                          │
                          ▼
  ┌──────────────────────────────────────────────────┐
  │               Fully connected layer(s)            │
  └──────────────────────────────────────────────────┘
```

First task group   Second task group   Third task group   ● ● ●   Auxiliary task group

N task groups

FIG. 3

FIG. 4A

FIG. 4B

FIG. 4C

FIG. 5A

FIG. 5B

FIG. 5C

FIG. 6A

FIG. 6B

FIG. 7

```
                          ┌─────────────┐
                          │    Start    │
                          └──────┬──────┘
                                 │
                                 ▼
   ┌──────────────────────────────────────────────────────┐
   │    Acquire electrocardiography data of subject        │─── S70
   └──────────────────────────┬───────────────────────────┘
                              │
                              ▼
   ┌──────────────────────────────────────────────────────┐
   │ Preprocess electrocardiography data to remove noise   │─── S71
   │ using at least one of low-pass filter, high-pass      │
   │ filter, or notch filter                               │
   └──────────────────────────┬───────────────────────────┘
                              │
                              ▼
   ┌──────────────────────────────────────────────────────┐
   │ Input electrocardiography data to extract ECG         │─── S72
   │ embedding vector including feature information of      │
   │ electrocardiography data                              │
   └──────────────────────────┬───────────────────────────┘
                              │
                              ▼
   ┌──────────────────────────────────────────────────────┐
   │ Input ECG embedding vector to generate cardiac strain │─── S73
   │ information about one or more task items               │
   └──────────────────────────┬───────────────────────────┘
                              │
                              ▼
   ┌──────────────────────────────────────────────────────┐
   │ Output cardiac strain information generated by cardiac│─── S74
   │ strain task processing unit                           │
   └──────────────────────────┬───────────────────────────┘
                              │
                              ▼
                       ┌─────────────┐
                       │     End     │
                       └─────────────┘
```

## INTERNATIONAL SEARCH REPORT

| International application No. |
| --- |
| **PCT/KR2024/000047** |

| | |
| --- | --- |
| **A.   CLASSIFICATION OF SUBJECT MATTER** | |
| **A61B 5/346**(2021.01)i; **A61B 5/00**(2006.01)i; **G16H 50/20**(2018.01)i; **G16H 50/30**(2018.01)i; **G16H 50/50**(2018.01)i; **G06N 3/08**(2006.01)i | |
| According to International Patent Classification (IPC) or to both national classification and IPC | |

| **B.    FIELDS SEARCHED** |
| --- |
| Minimum documentation searched (classification system followed by classification symbols) |
| A61B 5/346(2021.01); A61B 5/00(2006.01); A61B 5/0472(2006.01); G06F 19/00(2011.01); G06Q 50/22(2012.01); G16H 10/60(2018.01) |
| Documentation searched other than minimum documentation to the extent that such documents are included in the fields searched |
| Korean utility models and applications for utility models: IPC as above<br>Japanese utility models and applications for utility models: IPC as above |
| Electronic data base consulted during the international search (name of data base and, where practicable, search terms used) |
| eKOMPASS (KIPO internal) & keywords: 심전도 (ECG), 심근변형 (myocardial strain), 인코더 (encoder), 벡터 (vector), 태스크 (task), 인공지능 (artificial intelligence) |

| **C.    DOCUMENTS CONSIDERED TO BE RELEVANT** | | |
| --- | --- | --- |
| Category* | Citation of document, with indication, where appropriate, of the relevant passages | Relevant to claim No. |
| Y | KR 10-2022-0095009 A (SEOUL NATIONAL UNIVERSITY HOSPITAL) 06 July 2022 (2022-07-06)<br>See paragraphs [0145]-[0168] and [0192]-[0209]; and claim 11. | 1-21 |
| Y | KR 10-2321661 B1 (KOREA UNIVERSITY RESEARCH AND BUSINESS FOUNDATION) 05 November 2021 (2021-11-05)<br>See paragraph [0024]; and claims 1 and 2. | 1-21 |
| Y | KR 10-1855369 B1 (SIEMENS HEALTHCARE GMBH) 08 May 2018 (2018-05-08)<br>See paragraph [0032]; and figures 4 and 5. | 6-9 |
| A | WO 2020-161605 A1 (CARDIOLOGS TECHNOLOGIES SAS) 13 August 2020 (2020-08-13)<br>See entire document. | 1-21 |
| A | KR 10-1834051 B1 (HEARTFLOW, INC.) 02 March 2018 (2018-03-02)<br>See entire document. | 1-21 |

☐ Further documents are listed in the continuation of Box C.     ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **05 April 2024** | **05 April 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/000047**

| Patent document cited in search report | | | Publication date (day/month/year) | Patent family member(s) | | | Publication date (day/month/year) |
|---|---|---|---|---|---|---|---|
| KR | 10-2022-0095009 | A | 06 July 2022 | CN | 116782829 | A | 19 September 2023 |
| | | | | EP | 4272645 | A1 | 08 November 2023 |
| | | | | KR | 10-2022-0122588 | A | 02 September 2022 |
| | | | | KR | 10-2437594 | B1 | 29 August 2022 |
| | | | | WO | 2022-146057 | A1 | 07 July 2022 |
| KR | 10-2321661 | B1 | 05 November 2021 | KR | 10-2021-0154675 | A | 21 December 2021 |
| | | | | KR | 10-2346824 | B1 | 05 January 2022 |
| | | | | US | 2023-0282352 | A1 | 07 September 2023 |
| | | | | WO | 2021-251796 | A1 | 16 December 2021 |
| KR | 10-1855369 | B1 | 08 May 2018 | CN | 105474219 | A | 06 April 2016 |
| | | | | CN | 105474219 | B | 18 October 2019 |
| | | | | KR | 10-2016-0060657 | A | 30 May 2016 |
| | | | | US | 10733910 | B2 | 04 August 2020 |
| | | | | US | 2016-0210435 | A1 | 21 July 2016 |
| | | | | WO | 2015-031576 | A1 | 05 March 2015 |
| WO | 2020-161605 | A1 | 13 August 2020 | AU | 2018-320182 | A1 | 28 February 2019 |
| | | | | AU | 2018-320182 | B2 | 30 November 2023 |
| | | | | AU | 2020-218863 | A1 | 19 August 2021 |
| | | | | CA | 3072976 | A1 | 28 February 2019 |
| | | | | CA | 3128804 | A1 | 13 August 2020 |
| | | | | CN | 111433860 | A | 17 July 2020 |
| | | | | CN | 113613559 | A | 05 November 2021 |
| | | | | DK | 3673493 | T3 | 18 January 2021 |
| | | | | EP | 3367897 | A1 | 05 September 2018 |
| | | | | EP | 3367897 | B1 | 14 April 2021 |
| | | | | EP | 3673493 | A1 | 01 July 2020 |
| | | | | EP | 3673493 | B1 | 16 December 2020 |
| | | | | EP | 3826031 | A1 | 26 May 2021 |
| | | | | EP | 3878364 | A1 | 15 September 2021 |
| | | | | EP | 3920789 | A1 | 15 December 2021 |
| | | | | ES | 2860099 | T3 | 04 October 2021 |
| | | | | ES | 2869973 | T3 | 26 October 2021 |
| | | | | HU | E052862 | T2 | 28 May 2021 |
| | | | | HU | E054674 | T2 | 28 September 2021 |
| | | | | JP | 2020-531225 | A | 05 November 2020 |
| | | | | JP | 2022-523741 | A | 26 April 2022 |
| | | | | JP | 2023-097451 | A | 07 July 2023 |
| | | | | LT | 3673493 | T | 25 March 2021 |
| | | | | PL | 3673493 | T3 | 14 June 2021 |
| | | | | PT | 3367897 | T | 25 May 2021 |
| | | | | PT | 3673493 | T | 02 February 2021 |
| | | | | SG | 11202001229 | A | 30 March 2020 |
| | | | | SG | 11202108057 | A | 30 August 2021 |
| | | | | US | 10426364 | B2 | 01 October 2019 |
| | | | | US | 10758139 | B2 | 01 September 2020 |
| | | | | US | 10779744 | B2 | 22 September 2020 |
| | | | | US | 10827938 | B2 | 10 November 2020 |
| | | | | US | 10959660 | B2 | 30 March 2021 |
| | | | | US | 11134880 | B2 | 05 October 2021 |
| | | | | US | 11147500 | B2 | 19 October 2021 |

Form PCT/ISA/210 (patent family annex) (July 2022)

## INTERNATIONAL SEARCH REPORT
### Information on patent family members

International application No.

**PCT/KR2024/000047**

| Patent document cited in search report | | | Publication date (day/month/year) | Patent family member(s) | | | Publication date (day/month/year) |
|---|---|---|---|---|---|---|---|
| | | | | US | 11331034 | B2 | 17 May 2022 |
| | | | | US | 11672464 | B2 | 13 June 2023 |
| | | | | US | 11826150 | B2 | 28 November 2023 |
| | | | | US | 2017-0112401 | A1 | 27 April 2017 |
| | | | | US | 2019-0167143 | A1 | 06 June 2019 |
| | | | | US | 2019-0223739 | A1 | 25 July 2019 |
| | | | | US | 2019-0298204 | A1 | 03 October 2019 |
| | | | | US | 2020-0015694 | A1 | 16 January 2020 |
| | | | | US | 2020-0022604 | A1 | 23 January 2020 |
| | | | | US | 2021-0000365 | A1 | 07 January 2021 |
| | | | | US | 2021-0204860 | A1 | 08 July 2021 |
| | | | | US | 2022-0022799 | A1 | 27 January 2022 |
| | | | | US | 2022-0031223 | A1 | 03 February 2022 |
| | | | | US | 2022-0104750 | A1 | 07 April 2022 |
| | | | | US | 2022-0265199 | A1 | 25 August 2022 |
| | | | | WO | 2017-072250 | A1 | 04 May 2017 |
| | | | | WO | 2019-038435 | A1 | 28 February 2019 |
| | | | | WO | 2019-186439 | A1 | 03 October 2019 |
| KR | 10-1834051 | B1 | 02 March 2018 | AU | 2013-315960 | A1 | 19 March 2015 |
| | | | | AU | 2013-315960 | B2 | 14 April 2016 |
| | | | | AU | 2016-204888 | A1 | 28 July 2016 |
| | | | | AU | 2016-204888 | B2 | 12 July 2018 |
| | | | | CA | 2882543 | A1 | 20 March 2014 |
| | | | | CA | 2882543 | C | 12 September 2017 |
| | | | | CA | 2974349 | A1 | 20 March 2014 |
| | | | | CA | 2974349 | C | 01 June 2021 |
| | | | | CA | 3114366 | A1 | 20 March 2014 |
| | | | | CA | 3114366 | C | 28 March 2023 |
| | | | | CN | 104854592 | A | 19 August 2015 |
| | | | | CN | 104854592 | B | 14 September 2018 |
| | | | | CN | 108992059 | A | 14 December 2018 |
| | | | | CN | 108992059 | B | 07 July 2023 |
| | | | | EP | 2721543 | A2 | 23 April 2014 |
| | | | | EP | 2721543 | B1 | 23 August 2017 |
| | | | | EP | 3282381 | A1 | 14 February 2018 |
| | | | | EP | 3282381 | B1 | 17 November 2021 |
| | | | | EP | 3979259 | A1 | 06 April 2022 |
| | | | | JP | 2015-531264 | A | 02 November 2015 |
| | | | | JP | 2016-028747 | A | 03 March 2016 |
| | | | | JP | 2017-205605 | A | 24 November 2017 |
| | | | | JP | 2018-061883 | A | 19 April 2018 |
| | | | | JP | 2020-028774 | A | 27 February 2020 |
| | | | | JP | 5868554 | B2 | 24 February 2016 |
| | | | | JP | 6271492 | B2 | 31 January 2018 |
| | | | | JP | 6522175 | B2 | 29 May 2019 |
| | | | | JP | 7048561 | B2 | 05 April 2022 |
| | | | | KR | 10-2016-0031026 | A | 21 March 2016 |
| | | | | KR | 10-2021-0052589 | A | 10 May 2021 |
| | | | | KR | 10-2248498 | B1 | 06 May 2021 |
| | | | | KR | 10-2358996 | B1 | 08 February 2022 |

**INTERNATIONAL SEARCH REPORT**
Information on patent family members

International application No.

**PCT/KR2024/000047**

| Patent document cited in search report | Publication date (day/month/year) | Patent family member(s) | | | Publication date (day/month/year) |
|---|---|---|---|---|---|
| | | US | 10010255 | B2 | 03 July 2018 |
| | | US | 10398386 | B2 | 03 September 2019 |
| | | US | 10433740 | B2 | 08 October 2019 |
| | | US | 10561324 | B2 | 18 February 2020 |
| | | US | 10966619 | B2 | 06 April 2021 |
| | | US | 11013425 | B2 | 25 May 2021 |
| | | US | 11382569 | B2 | 12 July 2022 |
| | | US | 11399729 | B2 | 02 August 2022 |
| | | US | 2014-0073976 | A1 | 13 March 2014 |
| | | US | 2014-0073977 | A1 | 13 March 2014 |
| | | US | 2015-0245775 | A1 | 03 September 2015 |
| | | US | 2016-0310018 | A1 | 27 October 2016 |
| | | US | 2016-0310019 | A1 | 27 October 2016 |
| | | US | 2016-0310096 | A1 | 27 October 2016 |
| | | US | 2016-0317046 | A1 | 03 November 2016 |
| | | US | 2016-0317114 | A1 | 03 November 2016 |
| | | US | 2016-0321417 | A1 | 03 November 2016 |
| | | US | 2017-0281011 | A1 | 05 October 2017 |
| | | US | 2018-0235482 | A1 | 23 August 2018 |
| | | US | 2019-0336084 | A1 | 07 November 2019 |
| | | US | 2019-0380591 | A1 | 19 December 2019 |
| | | US | 2020-0359910 | A1 | 19 November 2020 |
| | | US | 2022-0322953 | A1 | 13 October 2022 |
| | | US | 9974453 | B2 | 22 May 2018 |
| | | WO | 2014-042899 | A2 | 20 March 2014 |
| | | WO | 2014-042899 | A3 | 17 July 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230000440

- KR 1020230194390